# EUROPÄISCHE PATENTSCHRIFT

(11) **EP 2 953 928 B1**
(45) Veröffentlichungstag und Bekanntmachung des Hinweises auf die Patenterteilung: **09.08.2017**
(21) Anmeldenummer: 14705308.6
(22) Anmeldetag: 05.02.2014
(51) Int. Cl.: C07C 263/20, C07C 265/14

(54) **VERFAHREN ZUR ABTRENNUNG EINES DURCH PHOSGENIERUNG EINES PRIMÄREN AMINS IN DER GASPHASE HERGESTELLTEN ISOCYANATS AUS DEM GASFÖRMIGEN ROHPRODUKT DER PHOSGENIERUNG**
METHOD FOR THE SEPARATION OF AN ISOCYANATE PRODUCED BY PHOSGENATION OF A PRIMARY AMINE IN THE GAS PHASE FROM THE GASEOUS RAW PRODUCT OF THE PHOSGENATION
PROCÉDÉ DESTINÉ À SÉPARER UN ISOCYANATE FABRIQUÉ PAR PHOSGÉNATION D'UNE AMINE PRIMAIRE EN PHASE GAZEUSE ET LE PRODUIT BRUT GAZEUX DE LA PHOSGÉNATION

(30) Priorität: 08.02.2013 EP 13154596
(43) Veröffentlichungstag der Anmeldung: 16.12.2015
(73) Patentinhaber: Covestro Deutschland AG, 51373 Leverkusen (DE)
(72) Erfinder: STEFFENS, Friedhelm, 51373 Leverkusen (DE); MAHR, Bastian, 51467 Bergisch Gladbach (DE)
(74) Vertreter: Levpat
(86) Internationale Anmeldenummer: PCT/EP2014/052260
(87) Internationale Veröffentlichungsnummer: WO 2014/122180

(56) Entgegenhaltungen:
- EP-A1- 1 403 248
- EP-B1- 1 935 875
- WO-A1-2010/063655

## Beschreibung

Die vorliegende Erfindung betrifft ein Verfahren zur Abtrennung eines durch Umsetzung eines primären Amins mit einem Überschuss an Phosgen in der Gasphase hergestellten Isocyanats aus dem in der Umsetzung erhaltenen gasförmigen Rohprodukt, bei dem
(i) das gasförmige Rohprodukt durch Inkontaktbringen mit einer Quenchflüssigkeit teilweise verflüssigt wird,
(ii) die in Schritt (i) erhaltene Gasphase teilweise kondensiert wird,
(iii) das in Schritt (ii) erhaltene Kondensat als Quenchflüssigkeit in Schritt (i) eingesetzt wird,
(iv) die in Schritt (ii) nicht kondensierten Anteile der Gasphase zumindest teilweise verflüssigt werden,
(v) die in Schritt (iv) erhaltene flüssige Phase ebenfalls als Quenchflüssigkeit in Schritt (i) eingesetzt wird, und
(vi) die in Schritt (i) erhaltene Flüssigphase ohne vorherigen Einsatz als Quenchflüssigkeit zum reinen Isocyanat aufgearbeitet wird.

Die Herstellung von Isocyanaten, insbesondere Diisocyanaten, in der Gasphase ist schon seit längerem im Stand der Technik beschrieben und wird industriell, insbesondere zur Herstellung von Toluylendiisocyanat, 1,6-Hexamethylendiisocyanat, Isophorondiisocyanat und Diisocyanatodicyclohexylmethan, genutzt. In allen Verfahren fällt ein gasförmiges Rohprodukt an, dass mindestens Isocyanat, Chlorwasserstoff und nicht umgesetztes Phosgen (Phosgen wird stets im Überschuss eingesetzt) umfasst, und das weiter aufgearbeitet werden muss, um das gewünschte Isocyanat in Reinform zu gewinnen.

Ein solches Verfahren ist beispielsweise in EP 0 289 840 B1 beschrieben. Die in einem Rohrreaktor gebildeten Diisocyanate sind bei den Reaktionstemperaturen von bis zu 500 °C thermisch nicht stabil. Eine rasche Abkühlung der Reaktionsgase nach der Phosgenierungsreaktion auf Temperaturen unter 150 °C ist daher notwendig, um die Bildung unerwünschter Nebenprodukte durch den thermischen Zerfall von Diisocyanat oder durch eine Weiterreaktion zu vermeiden. In EP 0 289 840 B1 oder EP 0749 958 B1 wird dazu das den Reaktionsraum kontinuierlich verlassende gasförmige Gemisch, welches u. a. Diisocyanat, Phosgen und Chlorwasserstoff enthält, in ein inertes Lösungsmittel, z. B. Dichlorbenzol, eingeleitet. Nachteilig bei diesem Verfahren ist, dass die Strömungsgeschwindigkeit, mit der das Gasgemisch durch das Lösungsmittelbad geleitet wird, relativ niedrig gewählt werden muss, da bei zu hohen Geschwindigkeiten Lösungsmittel und die darin gelösten Verbindungen mitgerissen würden. In einen nachfolgenden Schritt müssten die flüssigen Verbindungen vom Gas abgetrennt werden. Ein weiterer Nachteil ist, dass aufgrund der niedrigen Strömungsgeschwindigkeit und eines geringen Wärmeübergangs große Lösungsmittelbehälter eingesetzt werden müssen, um die Abkühlung zu erzielen.

Weiterhin sind Verfahren bekannt, die zur Abkühlung der Reaktionsgase Wärmeaustauscher einsetzen und/oder die Gase im Vakuum entspannen (DE 101 58 160 A1). Der Nachteil von Wärmeaustauschern liegt darin, dass wegen des schlechten Wärmeübergangs große Austauschflächen, und damit große Wärmeaustauscher, für eine effektive Kühlung benötigt werden. Außerdem kann es zu Ablagerungen von Feststoffen auf den verhältnismäßig kalten Flächen der Wärmeaustauscher durch Nebenreaktionen des Gasgemisches, wie z. B. Zersetzung, Polymerisation oder Ausfällung, kommen.

Im Verfahren gemäß der EP 1 761 483 B1 wird versucht, die Verweilzeit zwischen Reaktionsende und Abkühlzone dadurch zu verringern, dass sich zwischen der Reaktionszone und der Zone, in der der Reaktionsabbruch herbeigeführt wird, ein Bereich mit reduziertem Strömungsquerschnitt befindet.

Die Anmeldung WO2007/014936 A2, Verfahren zur Herstellung von Isocyanaten (in der Gasphase), beschreibt eine Quenchzone, in welcher durch Eindüsen einer Quenchflüssigkeit das gasförmige Rohprodukt rasch abgekühlt wird. In dieser Quenchzone wird das Reaktionsgemisch, das im Wesentlichen aus den Isocyanaten, Phosgen und Chlorwasserstoff besteht, intensiv mit der eingedüsten Flüssigkeit vermischt. Die Vermischung erfolgt derart, dass die Temperatur des Reaktionsgemisches ausgehend von 200 bis 570 °C auf 100 bis 200 °C abgesenkt wird und das im Reaktionsgemisch enthaltene Isocyanat durch Kondensation vollständig oder teilweise in die eingedüsten Flüssigkeitströpfchen übergeht, während das Phosgen und der Chlorwasserstoff im Wesentlichen vollständig in der Gasphase verbleiben. Als mögliche Quenchflüssigkeiten werden Lösungsmittel, Isocyanat-Gemische und Lösungsmittel-Isocyanat-Gemische genannt. Erwähnt wird das Eindüsen einer Quenchflüssigkeit zum Abkühlen des Reaktionsgemisches und selektiven Lösen des gebildeten Diisocyanats im Lösungsmittel, wobei eine erste Trennung in eine Flüssigphase und eine Gasphase mit überwiegend Phosgen und Chlorwasserstoff als Bestandteilen erfolgt. Die beiden Phasen werden danach einer entsprechenden Aufarbeitung zugeführt. Auf Optimierungsmöglichkeiten dieses Verfahrensschrittes wird nicht eingegangen.

Auch in WO 2011/003532 A1 wird ein Verfahren zum raschen Abkühlen des gasförmigen Reaktionsgemisches mittels Eindüsen einer Quenchflüssigkeit in das kontinuierlich aus der Reaktionszone in die nachgeschaltete Quenchzone strömende Gasgemisch offenbart.

Das Eindüsen von Quenchflüssigkeit mit Hilfe von mindestens zwei Sprühdüsen, welche am Eingang der Quenchzone angeordnet sind, wird in EP 1 403 248 B1 offenbart. Hierbei eignen sich als Quenchflüssigkeiten organische Lösungsmittel oder eine Mischung verschiedener organischer Lösungsmittel, welche mit dem gebildeten Diisocyanat nicht reagieren. Eine Lösung des gebildeten Diisocyanats in einem geeigneten organischen Lösungsmittel kann auch verwendet werden, was die Menge von eingesetztem Lösungsmittel reduziert. Der Durchmesser der Quenchzone kann größer oder kleiner sein als der Durchmesser der Reaktionszone. Das Quenchen der Reaktionsgase kann einstufig und auch mehrstufig erfolgen.

Optimiert wird dieses System gemäß der Lehre von EP 1 935 875 B1 dadurch, dass zum Abbruch der Reaktion das Reaktionsgemisch aus dem Reaktionsraum heraus durch eine Kühlstrecke geführt wird, in die hinein in zwei Zonen Flüssigkeiten eingedüst werden, sodass die direkte Kühlung in der Kühlstrecke einstufig (d. h. nur ein Kondensationsgemisch erhaltend) in zwei oder mehreren hintereinander geschalteten Kühlzonen erfolgt. Dabei wird mindestens in der zweiten Zone eine Kühlflüssigkeit eingesetzt, die das hergestellte Isocyanat in beträchtlichen Anteilen enthält (vgl. Patentanspruch 1, letzter Absatz). Das erzeugte Diisocyanat wird dabei in einem gemeinsamen Kondensationsgemisch erhalten. Dieses Gemisch wird vorzugsweise in einem Flüssigkeitssammelbehälter, angeordnet unterhalb der Kühlstrecke, aufgefangen. Dieses Kondensationsgemisch kann zur Abtrennung des hergestellten Isocyanats ausgeschleust oder, bevorzugt nach erfolgter Kühlung, teilweise auf eine oder mehrere Kühlzonen der Kühlstrecke zurückgeführt werden. Nachteilig an diesem Einsatz des flüssigen rohen Produktgemisches aus der Gasphasenphosgenierung kann eine eintretende Verschmutzung des beschriebenen Kühlers vor Eintritt in die Quenche sein. Ursache dafür können unerwünschte Nebenprodukte bzw. Polymerverbindungen aus der Phosgenierungsreaktion sein. Neben dem Kondensationsgemisch im Sammelbehälter wird nach der Kühlstrecke ein Gasstrom, enthaltend wenigstens Chlorwasserstoff, Phosgen, ggf. Lösungsmittel, und das hergestellte Isocyanat, erhalten. Dieser Gasstrom wird dem Sammelbehälter entnommen und einer Waschkolonne zugeführt und dort weitgehend von seinen Isocyanatanteilen befreit. Bevorzugt erfolgt diese Wäsche im Gegenstrom mit Lösungsmittel. Die so erhaltene Waschphase, bestehend aus Diisocyanat und zum überwiegenden Teil aus Lösungsmittel, wird in einer bevorzugten Ausführungsform als Quenchflüssigkeit der ersten Kühlzone der Kühlstrecke eingesetzt. Das Restgas aus der Waschkolonne besteht im Wesentlichen aus Phosgen, Chlorwasserstoff und Lösungsmittel. Diese Brüden verlassen die Kolonne über Kopf, wobei mittels Teilkondensation in einer bevorzugten Ausführungsform über zwei Kondensatoren mit unterschiedlichen Kühlmitteltemperaturen der Lösungsmittelanteil weitgehend zurückgehalten wird und auf die Kolonne als Partialkondensat zurückgeführt wird. Das danach erhaltene Restgas, das im Wesentlichen aus Phosgen, Chlorwasserstoff und Lösungsmittelresten besteht, wird anschließend in an sich bekannter Weise, wie z. B. in EP 1 849 767 B1 beschrieben, weiter behandelt.

In EP 1 935 876 A1 wird ebenfalls der Einsatz verschiedener geeigneter Quenchflüssigkeitsströme erwähnt. Dabei wird auch auf den Einsatz der Waschflüssigkeit aus der Gaswäsche der den Kondensatsammelbehälter nach der Quenche verlassenden Brüden als Quenchflüssigkeit hingewiesen.

Auf mehrere Kühlzonen in der Quench-Stufe weist auch EP 2 196 455 A1 hin. Auf den integrierten Verbund der Kühlzonen mehrerer Reaktoren mit einer Quench-Stufe wird hier erstmals hingewiesen.

WO 2010/063665 A1 verweist auf ein mögliches Problem der bisher genannten Quenche-Varianten. Wird zumindest ein Teil der Quenchflüssigkeit aus dem Sammelbehälter nach der Quenche, also das flüssige rohe Produktgemisch, entnommen, besteht die Möglichkeit, dass Feststoffe enthalten sein können, die die Quench-Düsen verstopfen können. Es werden verschiedene Techniken, wie z. B. Zentrifugieren, Abdestillieren des zur Quenche vorgesehenen Flüssigkeitsanteils oder Filtrieren beschrieben. Um die Temperatur des ausgewählten Quenche-Stromes für die gestellte Aufgabe einzustellen, kann der Strom mittels eines Wärmeaustauschers gekühlt, bzw. autgeheizt werden. Diese Schrift offenbart (S. 12, Z. 6 bis 9, S. 13, zweiter Absatz) verschiedene Quellen für das Quenchmedium: Einen aus dem der Quenche 3 nachgeschalteten Phasentrenner 9 abgezweigten Teilstrom 15 (der notwendigerweise auch in der Quenche verflüssigtes Isocyanat enthält), frisches Lösungsmittel 19, einen Teil der im Phasentrenner gewonnenen Flüssigphase 13 sowie einen Teilstrom des zweiphasigen Produktstroms 7. Die Schrift offenbart keine Ausführungsform, in welcher das Quenchmedium aus der im Phasentrenner 9 gewonnenen Gasphase 11 erhalten und auf eine Rückführung von in der Quenche verflüssigtem Isocyanat vollständig verzichtet wird.

In WO 2010/115908 A2 wird eine spezielle Ausführungsform der Quenche offenbart. Um Folgereaktionen des Reaktionsgases bei bzw. nach der Quenchestufe zu verhindern, werden die Quenchedüsen und deren Anordnung derart konzipiert, dass eine weitgehend vollständige Benetzung der Wandung im Quenchebereich erfolgt. Damit wird das gesamte Reaktionsgemisch erfasst. Als Quenchflüssigkeiten werden Lösungsmittel sowie Gemische mit Isocyanat bzw. Rohgemisch der Phosgenierreaktion, ggf. nach Partikelentfernung, vorgeschlagen.

EP 2 463 273 A1 offenbart eine Prozessvariante für Isocyanatkonzentrationen von größer als 70 Massen-% im die Quenchzone verlassenden flüssigen Sumpfprodukt. Der die Quenchzone gasförmig verlassende Strom wird ohne Durchlaufen einer Waschkolonne direkt in einen mantelgekühlten Kondensator geleitet. Der verbleibende Gasstrom wird direkt der Phosgenrückgewinnung zugeführt. Trotz der hohen Temperatur und hohen Isocyanatkonzentration im flüssigen Sumpfprodukt der Quenchzone werden über Isocyanat-Restgehalte im verbleibenden Gasstrom keine Aussagen gemacht. Der Kondensatstrom wird mit dem Kondensat des Dampfstroms, der durch Entspannung des flüssigen Sumpfproduktes aus der Quenchzone entsteht, vereinigt und als Quenchflüssigkeit zurückgeführt.

Die bisherigen Schriften befassten sich im Quenche-Bereich der Gasphasenphosgenierung von Diaminen im Wesentlichen mit der Optimierung der eigentlichen Quenche-Systeme. Bis auf wenige Ausnahmen wurden die mit der Quenche in Verbindung stehenden peripheren Systeme nicht betrachtet. Die Ausnahmen sind zum Beispiel, wie oben zitiert, der Einsatz von Kondensationsgemisch aus dem Flüssigkeitssammelbehälter der Quenche bzw. des Kondensats der durch Entspannungsverdampfung des Kondensationsgemischs entstandenen Dampfstroms, oder der Einsatz der Waschflüssigkeit aus der Gaswäsche der den Kondensatsammelbehälter nach der Quenche verlassenden Brüden als Quenchflüssigkeit. Durch die Verwendung von Kondensationsgemisch aus dem Flüssigkeitssammelbehälter der Quenche wird die erforderliche Lösungsmittelmenge zur Abkühlung des Reaktionsgases verringert, indem von außen zugeführte Quenchflüssigkeit durch in der Gasphasenphosgenierung erzeugtes Diisocyanat und in dem Kondensat bereits enthaltenes Lösungsmittel ersetzt wird. Ebenso lässt sich der Gesamtanfall an Quencheflüssigkeit durch den teilweisen Einsatz von Waschflüssigkeit aus der Gaswäsche der den Kondensatsammelbehälter nach der Quenche verlassenden Brüden reduzieren. Beide Verfahrensweisen führen zur Verringerung des Lösungsmittelkreislaufs im Gesamtverfahren und tragen somit bei erfolgreichem Einsatz zur Reduzierung des Energieverbrauchs bei und können gegebenenfalls einen Beitrag zur Kostenreduzierung im Apparatebereich leisten.

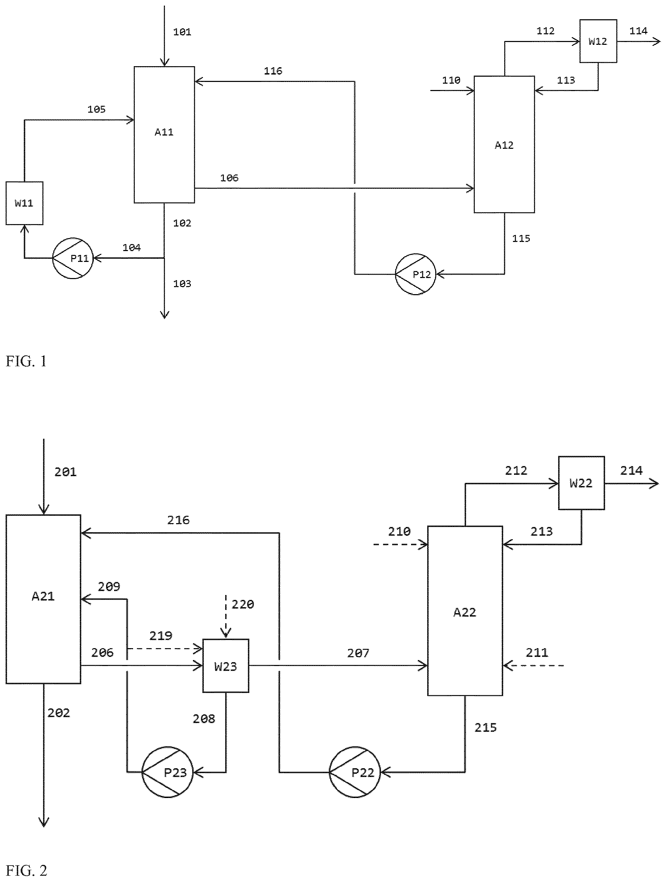

Ein typisches Verfahren des Standes der Technik sei beispielhaft anhand der ersten Abbildung (Fig. 1) erläutert:
Das gasförmige Rohprodukt (101), vorwiegend bestehend aus Isocyanat, Chlorwasserstoff und überstöchiometrisch eingesetztem Phosgen, wird in der Quenche (A11) durch Eindüsen von Quenchflüssigkeit (105 und 116) schnell abgekühlt, um unerwünschte Folgereaktionen zu vermeiden. Der die Quenche flüssig verlassende Strom (102), enthaltend vor allem Isocyanat und Quenchflüssigkeit, wird zum kleineren Teil zur Produktreinigung geleitet und zum größeren Teil über den Quenchekühler (W11) als Quencheflüssigkeit zur Quenche zurückgeführt. Aufgrund des großen Kreislaufstroms muss eine entsprechend groß dimensionierte Pumpe (P11) installiert werden, und eine thermische Belastung des Produkts durch mehrfaches in Kontakt bringen mit dem heißen Gasstrom aus der Reaktion in Kauf genommen werden, was zu Ausbeuteverlusten und erhöhtem Aufarbeitungsaufwand führt. In der Reaktions- oder Quenchezone entstehende oder darin eingetragene Feststoffe sowie Schwersieder gelangen mit dem flüssigen Quencheprodukt in den Quenchekühler (W11) und die Quenchedüsen und können dort Verschmutzungen verursachen.

Der die Quenche gasförmig verlassende Stoffstrom (106), enthaltend vor allem verdampfte Quenchflüssigkeit, Chlorwasserstoff und Phosgen, wird der Waschkolonne (A12) zugeleitet, um Restgehalte an Isocyanat möglichst weitgehend aus dem Brüdenstrom zu entfernen. Je größer der Gehalt an Isocyanaten im zuge führten Stoffstrom (106) ist, desto höher muss der am Kopf der Waschkolonne zugeführte Waschflüssigkeitsstrom (Lösungsmittel) gewählt werden, und desto mehr Trennstufen werden für eine zuverlässige Rückhaltung benötigt. Der Waschflüssigkeitsstrom setzt sich aus dem Kondensat (113) des Kondensators (W12) und zusätzlichem - ggf. Leichtsieder wie Phosgen enthaltendem, aber praktisch Isocyanat-freiem - Lösungsmittel (110) zusammen. Der praktisch Isocyanat-freie Brüdenstrom (114) enthält vor allem Phosgen und Chlorwasserstoff. Der flüssige Sumpfablauf (115) enthält vor allem Lösungsmittel und wird zweckmäßigerweise als Quencheflüssigkeit der Quenche zugeführt.

Die Einstellung der Temperatur der Stoffströme am Austritt des Quenche-Schrittes muss unter Abwägung gegenläufiger Ziele erfolgen, u. a. zwischen geringer thermischer Produktbelastung und geringem Lösungsmittel- und Isocyanatanteil im zu reinigenden und zu recyclierenden Brüdenstrom einerseits *(niedrige Temperatur ist positiv)* und geringer Carbaminsäurechlorid-Bildung und geringem Energiebedarf in der Produktaufreinigung andererseits *(hohe Temperatur ist positiv).* Die Temperatur der Stoffströme am Austritt der Quenchzones wird bei vorgegebenem Gasstrom aus der Reaktionszone durch die Menge, Temperatur und Zusammensetzung der Quenchflüssigkeits-Ströme bestimmt.

Aufbauend auf diesem Stand der Technik bestand ein Bedarf nach einer weiteren Optimierung der Quenche eines gasförmigen Isocyanat-Rohprodukts. Insbesondere sollten die Prozessabschnitte (a) Überführung des flüssigen rohen Produktgemisches aus der Quenche in die weiteren Aufarbeitungsstufen, (b) Behandlung der in der Quenche erhaltenen Gasphase (Brüden) einschließlich der Gaswäsche und (c) Versorgung der Quench-Stufe vor dem Hintergrund niedriger Betriebs- und Apparatekosten bei hoher Verfügbarkeit und einfacher, zuverlässiger Regelbarkeit weiter optimiert werden.

Diesem Bedarf Rechnung tragend ist ein Gegenstand der vorliegenden Erfindung ein Verfahren zur Abtrennung eines durch Umsetzung eines primären Amins mit einem Überschuss an Phosgen in der Gasphase hergestellten Isocyanats aus dem in der Umsetzung erhaltenen gasförmigen Rohprodukt, bei dem
(i) das gasförmige Rohprodukt (201), das mindestens Isocyanat, Chlorwasserstoff und nicht umgesetztes Phosgen umfasst, durch Inkontaktbringen mit, bevorzugt durch Eindüsen, mindestens einer Quenchflüssigkeit in eine mindestens Quenchflüssigkeit und Isocyanat umfassende Flüssigphase (202) und in eine mindestens Chlorwasserstoff und Phosgen umfassende Gasphase (206) überführt wird (sog. "Quenche" des gasförmigen Rohprodukts),
(ii) die in Schritt (i) erhaltene Gasphase (206) teilweise kondensiert wird,
(iii) das in Schritt (ii) erhaltene Kondensat (208) als Quenchflüssigkeit in Schritt (i) eingesetzt wird,
(iv) die in Schritt (ii) nicht kondensierten Anteile (207) der Gasphase (206) zumindest teilweise unter Erhalt einer flüssigen Phase (215) verflüssigt werden,
(v) die in Schritt (iv) erhaltene flüssige Phase (215) ebenfalls als Quenchflüssigkeit in Schritt (i) eingesetzt wird, und
(vi) die in Schritt (i) erhaltene Flüssigphase (202) ohne vorherigen Einsatz als Quenchflüssigkeit zum reinen Isocyanat aufgearbeitet wird.

Das Wort *"ein"* im Zusammenhang mit zählbaren Größen ist im Rahmen der vorliegenden Erfindung nur dann als Zahlwort zu verstehen, wenn dies ausdrücklich gesagt wird (z. B. durch den Ausdruck *"genau ein"*)*.* Wenn im Folgenden beispielsweise von *"einem Kondensator"* gesprochen wird, ist das Wort *"ein"* lediglich als unbestimmter Artikel und nicht als Zahlwort aufzufassen, es ist also damit auch eine Ausführungsform umfasst, in der zwei oder mehr Kondensatoren in Reihe geschaltet sind.

Die Einschränkung *"ohne vorherigen Einsatz als Quenchflüssigkeit"* in Schritt (vi) des erfindungsgemäßen Verfahrens bedeutet, dass in Schritt (i) verflüssigtes Isocyanat nicht Bestandteil der in Schritt (i) eingesetzten Quenchflüssigkeit(en) ist, d. h. es findet im Gegensatz zum Stand der Technik keine partielle Rückführung des verflüssigten Isocyanats in die "Quenche" des gasförmigen Rohprodukts (201) statt.

Nachstehend werden Ausführungsformen des erfindungsgemäßen Verfahrens näher beschrieben. Verschiedene Ausführungsformen können dabei beliebig miteinander kombiniert werden, sofern sich für den Fachmann aus dem Kontext nicht eindeutig das Gegenteil ergibt. Bezugszeichen mit einer "2" als erster Ziffer beziehen sich auf die zweite Abbildung (Fig. 2), in der eine bevorzugte Ausgestaltung des erfindungsgemäßen Verfahrens schematisch dargestellt ist.

Die **Umsetzung eines primären Amins mit einem Überschuss an Phosgen in der Gasphase** zur Gewinnung des das entsprechende Isocyanat enthaltenden gasförmigen Rohprodukts (201) kann grundsätzlich nach einem beliebigen aus dem Stand der Technik bekannten Verfahren zur Gasphasenphosgenierung erfolgen. Beispiele für geeignete Gasphasenphosgenierungs-Verfahren sind in EP 0 570 799 A1, EP 1 555 258 A1, EP 1 526 129 A1 und DE 101 61 384 A1, sowie insbesondere für aliphatische Isocyanate in EP 0 289 840 B1 und EP 1 754 698 B1, beschrieben.

Geeignete *primäre Zeine* sind dabei insbesondere die Isomeren des Toluylendiamins (nachfolgend TDA), die Isomeren des Diphenylmethandiamins (nachfolgend MDA), 1,6-Hexamethylendiamin (nachfolgend HDA), die Isomeren des Isophorendiamins (nachfolgend IDPA) und die Isomeren des Diaminodicyclohexylmethans (nachfolgend H12-MDA). Besonders bevorzugt ist TDA, wobei die genaue jeweils vorliegende Isomerenzusammensetzung für das erfindungsgemäße Verfahren nicht von Belang ist. Üblicherweise umfasst TDA, welches bevorzugt eingesetzt wird, 78 Massen-% bis 82 Massen-% 2,4-TDA und 18 Massen-% bis 22 Massen-% 2,6-TDA, bezogen auf die Gesamtmasse der 2,4- und 2,6-TDA-Isomeren. Bezogen auf die Gesamtmasse des TDA machen dabei die 2,4- und 2,6-TDA-Isomeren bevorzugt in Summe von 95,0 Massen-% bis 100 Massen-%, besonders bevorzugt von 98,0 Massen-% bis 100 Massen-%, aus.

Das primäre Amin wird mit Phosgen umgesetzt. Dabei ist eine kontinuierliche wie auch diskontinuierliche Betriebsweise möglich. Bevorzugt ist eine Phosgenierung in kontinuierlicher Betriebsweise. Hierbei erfolgt die Umsetzung bevorzugt bei einer Temperatur von 200 °C bis 600 °C, bevorzugt von 300 °C bis 500 °C, und einem absoluten Druck von 150mbar bis 10 bar, bevorzugt von 1,0 bar bis 3,0 bar. Der molare Überschuss an Phosgen beträgt bevorzugt 20 % bis 400 % der Theorie.

**Schritt (i)** des erfindungsgemäßen Verfahrens, die rasche Abkühlung und teilweise Verflüssigung ("Quenche") des gasförmigen Rohprodukts (201) der Phosgenierung durch Inkontaktbringen mit, bevorzugt durch Eindüsen, einer Quenchflüssigkeit, kann in einem beliebigen aus dem Stand der Technik bekannten Apparat (A21) erfolgen. Geeignete Ausführungsformen sind beispielsweise in EP 1 403 248 A1, siehe insbesondere die Zeichnungen mit den zugehörigen Erläuterungen in den Absätzen [0017] bis [0019], und EP 1 935 875 A1, siehe insbesondere die Absätze [0015] bis [0022] und [0033] bis [0045], beschrieben, wobei im Rahmen der vorliegenden Erfindung jedoch zu beachten ist, dass in Schritt (i) verflüssigtes Isocyanat aus Strom 202 nicht Bestandteil der Quenchflüssigkeit ist (vgl. Schritt (vi) der vorliegenden Erfindung).

Neben den Quenchflüssigkeiten aus Schritt (iii) und Schritt (v) (siehe unten für weitere Details) kann zusätzlich ein frischer Strom eines organischen Lösungsmittels als Quenchflüssigkeit eingesetzt werden (in Fig. 2 nicht gezeigt). Bevorzugte Lösungsmittel sind chlorierte aromatische Kohlenwasserstoffe, wie beispielsweise Chlorbenzol, o-Dichlorbenzol, p-Dichlorbenzol, Trichlorbenzole, die entsprechenden Chlortoluole oder Chlorxylole, Chlorethylbenzol, Monochlordiphenyl, α- bzw. β-Naphthylchlorid, Benzoesäureethylester, Phthalsäuredialkylester, Diisodiethylphthalat, Toluol und Xylole. Besonders bevorzugte Lösungsmittel sind Chlorbenzol und die Isomeren des Dichlorbenols, außerordentlich besonders bevorzugt ist o-Dichlorbenzol. Dieser zusätzliche Strom an frischem organischem Lösungsmittel hat bevorzugt eine Temperatur von 40 °C bis 150 °C. Erfindungsgemäß ausgeschlossen ist jedoch eine auch nur teilweise Rückführung der Flüssigphase 202 als Quenchflüssigkeit (vgl. Schritt (vi) der vorliegenden Erfindung). Hierdurch wird erreicht, dass die eingesetzte(n) Quenchflüssikeit(en) allenfalls unbedeutende Mengen an Isocyanat (aus geringen Anteilen in Schritt (i) nicht verflüssigten Isocyanats, welche mit dem Gasstrom 206 die Quenche A21 verlassen) enthalten.

In Schritt (i) wird auf diese Weise eine mindestens Quenchflüssigkeit und Isocyanat umfassende Flüssigphase (202) und in eine mindestens Chlorwasserstoff und Phosgen umfassende Gasphase (206) erhalten.

**Schritt (ii)** des erfindungsgemäßen Verfahrens, die teilweise Kondensation der in Schritt (i) erhaltenen Gasphase (206), kann in einem beliebigen aus dem Stand der Technik bekannten Kondensator (W23) durchgeführt werden. Der Kondensator (W23) kann mehrstufig ausgeführt werden, z. B. um verschiedene Kühlmedien zu nutzen und / oder mehrere Prozesskondensatqualitäten zu erhalten, die ggf. an unterschiedliche Stellen im Prozess zurückgeführt werden können. Der Kondensator (W23) wird bevorzugt als indirekter Kühler mit Flüssigkeitsberieselung ausgeführt. Eine mögliche Ausführungsform ist beispielsweise ein Rieselfilmkondensator, bei dem ein Teil des Kondensats als Berieselungsflüssigkeit auf denselben Kondensator zurückgeführt wird. Die Gasphase (206) enthält neben Chlorwasserstoff und Phosgen im Allgemeinen auch noch Restmengen an Isocyanat und Quenchflüssigkeit, da die Trennung dieser von Phosgen und Chlorwasserstoff in Schritt (i) in der Regel nicht vollständig gelingt. In dem in Schritt (ii) eingesetzten Kondensator wird nun der überwiegende Teil der in der Gasphase (206) verbliebenen Quenchflüssigkeit und des Isocyanats kondensiert. Dies geschieht durch Abkühlung der Gasphase (206), die bevorzugt mit einer Temperatur von 120 °C bis 250 °C in den Kondensator geleitet wird, auf eine Temperatur von bevorzugt 40 °C bis 170 °C. Schritt (ii) wird bevorzugt zur Gewinnung von Dampf, bevorzugt Niederdruckdampf von 6 bar, genutzt.

Das entstehende Kondensat (208) enthält vor allem Lösungsmittel (aus der Quenchflüssigkeit) und eignet sich daher besonders für die Rückführung als Quencheflüssigkeit (209). Da es sich um das Kondensat aus einem dampfförmigen Strom handelt, ist der Anteil an Schwersiedern und die Gefahr von Feststoffanteilen sehr viel geringer als im flüssigen rohen Produktgemisch (202). Die Verschmutzungsneigung in Wärmetauscher (W23), Pumpe (P23), den Quenchedüsen für Strom (209) und zugehörigen Rohrleitungen ist also wesentlich geringer als eine mögliche Verschmutzungsneigung in den vergleichbaren, flüssiges rohes Produktgemisch führenden Anlagenteilen im Stand der Technik (Pumpe P11, Wärmeaustauscher W11, Quenchedüsen für Strom 105, zugehörige Rohrleitungen).

Zusätzlich zu der in Schritt (i) erhaltenen Gasphase (206) kann/können auch
(a) ein Teil (219) des in Schritt (ii) erhaltenen Kondensats (208) oder
(b) ein Lösungsmittelstrom (220) oder
(c) Strom (a) (219) und Strom (b) (220),
in den Kondensator (W23) geleitet werden. Hierdurch wird die Kondensation der Brüden (206) erleichtert, Wärmeübertragungsflächen gleichmäßig benetzt, Anhaftungen vermieden bzw. abgespült und/oder eine Absorptionswirkung erzielt.

Der Kondensator (W23) wird prozessseitig bevorzugt im Gleichstrom von Dampf- und Flüssigphase betrieben, um hohe Dampfgeschwindigkeiten und kleine Apparatedimensionen zu ermöglichen. Er kann aber auch im Gegenstrom ausgeführt werden, insbesondere um mehr als eine theoretische Trennstufe zu erreichen, um den Anteil an Isocyanat im austretenden Dampfstrom (207) zu minimieren.

Das in Schritt (iv) erhaltene Kondensat (208) wird aus dem Kondensator abgezogen, erforderlichenfalls mittels einer Pumpe (P23) gefördert, und in **Schritt (iii)** in der Quenchstufe (Schritt (i)) als Quenchflüssigkeit (209) verwendet. Bei geeigneter Apparateaufstellung (Gewährleistung eines freien Flüssigkeitsablaufs) kann die Pumpe entfallen. Erforderlichenfalls kann der Strom (208) mit Hilfe eines zusätzlichen Kühlmediums weiter gekühlt werden, bevor er als Quenchflüssigkeit (209) eingesetzt wird. Die Quenchflüssigkeit (209) wird bevorzugt mit einer Temperatur von 30 °C bis 170 °C mit dem gasförmigen Rohproduktstrom (201) in Kontakt gebracht, bevorzugt in diesen eingedüst. Der Einsatz des Kondensats (208) als Quenchflüssigkeit ersetzt die Quenchflüssigkeit (105) aus dem Stand der Technik. Mit anderen Worten, im erfindungsgemäßen Verfahren ist es nicht erforderlich, beträchtliche Teile des in der Quenchzone (A21) verflüssigten Rohprodukts als Quenchflüssigkeit einzusetzen. Die weiter oben beschriebenen, damit verbundenen Nachteile des Standes der Technik entfallen daher. Insbesondere kann auf den Quench-Kühler (W11) mit potenzieller Verschmutzungstendenz sowie die dazugehörende Kreislaufpumpe (P11) verzichtet werden. Durch den vollständigen Verzicht auf Rezyklierung des in der Quenchzone (A21) verflüssigten Rohproduktgemischs oder des Kondensats des durch Entspannungsverdampfung des flüssigen rohen Produktgemischs entstehenden (deutlich Isocyanat-haltigen) Dampfs in die Quenche wird die Neigung zur Bildung von Anbackungen in Rohrleitungen und der Quenchzone selbst sowie die Produktzersetzung reduziert.

**Schritt (iv)** des erfindungsgemäßen Verfahrens, die zumindest teilweise Verflüssigung der in Schritt (ii) nicht kondensierten Anteile (207) der Gasphase (206), wird bevorzugt in einer Waschkolonne (A22) mit mindestens einer Trennstufe durchgeführt, wobei der Waschkolonne (A22) mindestens ein Lösungsmittelstrom als Waschflüssigkeit zugeführt wird. Besonders bevorzugt wird der gasförmige Kopfstrom (212) der Waschkolonne (A22) in einem Kondensator (W22) kondensiert und das so erhaltene Kondensat (213) als zusätzliche Waschflüssigkeit in die Waschkolonne (A22) zurückgeführt. Geeignete Waschkolonnen (A22) für Schritt (iv) sind beispielsweise beschrieben in Perry's Chemical Engineers' Handbook, 7th Edition, McGraw-Hill, Kapitel 14 "Gas Absorption and Gas-Liquid System Design". Bevorzugte Lösungsmittel für Schritt (iv) sind chlorierte aromatische Kohlenwasserstoffe, wie beispielsweise Chlorbenzol, o-Dichlorbenzol, p-Dichlorbenzol, Trichlorbenzole, die entsprechenden Chlortoluole oder Chlorxylole, Chlorethylbenzol, Monochlordiphenyl, α- bzw. β-Naphthylchlorid, Benzoesäureethylester, Phthalsäuredialkylester, Diisodiethylphthalat, Toluol und Xylole. Besonders bevorzugte Lösungsmittel sind Chlorbenzol und die Isomeren des Dichlorbenzols, außerordentlich besonders bevorzugt ist o-Dichlorbenzol. Der mindestens eine Lösungsmittelstrom kann aus frischem Lösungsmittel bestehen. Es ist jedoch auch möglich, im Verfahren rezyklierte Lösungsmittelhaltige Ströme einzusetzen.

In einer Ausführungsform wird der Waschkolonne (A22) genau ein (von (213) verschiedener) Lösungsmittelstrom zugeführt.

In einer weiteren Ausführungsform wird ein Lösungsmittelstrom (210) oberhalb der Trennstufe der Waschkolonne (A22), bei Vorhandensein mehrerer Trennstufe oberhalb der obersten Trennstufe, zugeführt. Wenn als Lösungsmittelstrom (210) nicht frisches Lösungsmittel, sondern ein rezyklierter Lösungsmittel-haltiger Strom eingesetzt werden soll, ist darauf zu achten, dass dieser das abzutrennende Isocyanat in einem Massenanteil von maximal 100 ppm, bezogen auf die Gesamtmasse des Lösungsmittelstroms (210), enthält, um die Bildung von Zersetzungsprodukten zu vermeiden.

Alternativ zu oder in Kombination mit dieser Ausführungsform kann ein Lösungsmittelstrom (211) unterhalb der Trennstufe der Waschkolonne (A22), bei Vorhandensein mehrerer Trennstufen unterhalb der untersten Trennstufe, zugeführt werden. Wenn als Lösungsmittelstrom (211) nicht frisches Lösungsmittel, sondern ein rezyklierter Lösungsmittel-haltiger Strom eingesetzt werden soll, so sind hier die Anforderungen an den maximalen Isocyanat-Gehalt weniger streng als im Fall von Strom (210). Aufgrund des direkten Beitrags von Strom (210) zum Dampf-Flüssigkeits-Gleichwicht mit dem dampfförmigen Kopfproduktstrom der Waschkolonne, der weitgehend Isocyanat-frei sein muss, können in Strom (210) nur sehr viel geringere Isocyanat-Anteile zugelassen werden als in Strom (211). Daher kann der Lösungsmittelstrom (211) bis zu 20 Massen-% Isocyanat, bezogen auf die Gesamtmasse des Lösungsmittelstroms (211), enthalten.

Durch den Einsatz des Kondensators in Schritt (ii) im erfindungsgemäßen Verfahren wird im Unterschied zum Stand der Technik bewirkt, dass der der Waschkolonne (A22) zugeführte Volumenstrom vergleichsweise gering ist (nämlich nur 30 % bis 80 % des entsprechenden Gasstroms ohne Schritt (ii)), erheblich weniger Isocyanat enthält (nämlich bevorzugt 0 Massen-% bis 5 Massen-%, besonders bevorzugt 10ppm bis 0,5 Massen-%, jeweils bezogen auf die Gesamtmasse des Stroms 206), und eine geringere Temperatur (nämlich bevorzugt 40 °C bis 170 °C) aufweist. Wird die Kondensationstemperatur im Kondensator W23 geeignet gewählt (z. B. 60 °C), wird mit Hilfe von W23 gegenüber dem in Fig. 1 gezeigten Stand der Technik der Brüdenstrom zur Waschkolonne (207 gegenüber 106) im Volumen um ca. 55 % verringert und bezüglich der enthaltenen Isocyanatmenge etwa um den Faktor 1000 reduziert.

Infolgedessen kann im erfindungsgemäßen Schritt (iv) mit einer Waschkolonne geringen Durchmessers und mit einer geringen Trennstufenzahl gearbeitet werden. Die erniedrigte Temperatur und der damit verbundene geringere Gehalt an Quenchflüssigkeit der zur Waschkolonne geleiteten Gasphase macht darüber hinaus eine geringere Leistung des Kondensators der Waschkolonne erforderlich. Die geringeren oben genannten Apparatedimensionen erhöhen die Wirtschaftlichkeit des Verfahrens. Die vom Kondensator (W22) der Waschkolonne in den Kondensator (W23) von Schritt (ii) verschobene Kühlleistung kann aufgrund des höheren Kondensationstemperaturniveaus zur Gewinnung von Dampf, bevorzugt Niederdruckdampf von 6 bar, genutzt werden.

Im Vergleich zum Stand der Technik muss in der Waschkolonne (A22) weniger Waschflüssigkeit eingesetzt werden, da der zugeführte Brüdenstrom (207) bereits weniger Isocyanat enthält. Gemäß dem Stand der Technik wird die gesamte Waschflüssigkeit über den Sumpf der Waschkolonne in die Quenchzone geführt und bestimmt auf diese Weise den Lösungsmittelgehalt des am Sumpf der Quenchzone entnommenen verflüssigten rohen Produktgemischs. Eine geringere Menge an Waschflüssigkeit ermöglicht also im erfindungsgemäßen Verfahren einen geringeren Lösungsmittelgehalt im flüssigen rohen Produktgemisch und damit einen deutlich geringeren Aufarbeitungsaufwand in Schritt (vi), ohne dass die Reinigungswirkung (also die Verringerung des Isocyanat-Gehalts des Gasstroms) von Schritt (vi) gegenüber dem Stand der Technik verringert wird. Durch das erfindungsgemäße Verfahren verringern sich die Menge des Lösungsmittelkreislaufs und die benötigte elektrische Pumpenleistung, was sich in Energieeinsparung ausdrückt.

Neben der flüssigen Phase (215) fällt in Schritt (iv) auch ein gasförmiger Strom (214) an. Dieser gasförmige Strom besteht im Wesentlichen aus Chlorwasserstoffgas, stöchiometrisch überschüssigem Phosgen, weiteren Gasen, wie zum Beispiel Stickstoff und Kohlenmonoxid, und geringfügigen Mengen an Lösungsmittel. Dieser gasförmige Produktstrom wird bevorzugt einer weiteren Aufarbeitung zugeführt, wo in der Regel Lösungsmittel, überschüssiges Phosgen und entstandenes Chlorwasserstoffgas voneinander getrennt werden. Lösungsmittel und überschüssiges Phosgen werden (getrennt voneinander) aus wirtschaftlichen Gründen bevorzugt wieder der Reaktion zugeführt. Der Chlorwasserstoff kann verschiedenen Einsatzmöglichkeiten zugeführt werden, wie zum Beispiel einer Oxychlorierung von Ethylen zu Ethylendichlorid oder einem Recyclingverfahren, welches Chlor in den Isocyanatprozess wieder zurückführt. Zu diesen Recyclingverfahren zählen die katalytische Oxidation von Chlorwasserstoff, z. B. nach dem Deacon-Verfahren, die Elektrolyse von gasförmigem Chlorwasserstoff sowie die Elektrolyse einer wässrigen Lösung von Chlorwasserstoff (Salzsäure).

Die in Schritt (iv) erhaltene flüssige Phase (215) wird erforderlichenfalls mittels einer Pumpe (P22) gefördert und in **Schritt (v)** ebenfalls als Quenchflüssigkeit (216) in Schritt (i) eingesetzt. Bei geeigneter Apparateaufstellung (Gewährleistung eines freien Flüssigkeitsablaufs) kann die Pumpe entfallen. Erforderlichenfalls kann der Strom (215) mit Hilfe eines zusätzlichen Kühlmediums weiter gekühlt werden, bevor er als Quenchflüssigkeit (216) eingesetzt wird. Die Quenchflüssigkeit (216) wird bevorzugt mit einer Temperatur von 30 °C bis 150 °C mit dem gasförmigen Rohproduktstrom (201) in Kontakt gebracht, bevorzugt eingedüst. In einer Ausführungsform werden also die Quenchflüssigkeit des Schritts (iii) und die Quenchflüssigkeit des Schritts (v) in Schritt (i) getrennt voneinander (209, 216) in das gasförmigen Rohprodukt der Phosgenierung (201) eingebracht, bevorzugt eingedüst. Bevorzugt wird dabei die Quenchflüssigkeit (216) oberhalb der Quenchflüssigkeit von Schritt (iii) (209) in den gasförmigen Rohproduktstrom (201) eingebracht, bevorzugt eingedüst. Denn dadurch, dass im erfindungsgemäßen Verfahren das in den Brüden (206) enthaltene Isocyanat zu einem großen Teil bereits im Kondensator (W23) abgetrennt wird, sinkt, wie oben beschrieben, der Isocyanatanteil der in Schritt (iv) erhaltenen flüssigen Phase (215), die als Quenchflüssigkeit (216) eingesetzt wird. Da Isocyanat in der Quenchflüssigkeit insbesondere bei den hohen Temperaturen im obersten Teil der Quenchzone zu vermehrter unerwünschter Nebenproduktbildung führt, ist es also vorteilhaft, Strom (216) in der Quenchezone getrennt und bezüglich des Gasstroms stromaufwärts von Strom (209) zuzuführen. Gegenüber dem Stand der Technik (Fig. 1) ermöglicht der gegenüber (116) reduzierte Isocyanatanteil von (216) im erfindunggemäßen Verfahren eine vorteilhafte geringere Nebenproduktbildung. Es ist jedoch prinzipiell auch möglich (in Fig. 2 nicht gezeigt), die Ströme (208) und (215) zu vereinigen und gemeinsam als einen Quenchflüssigkeits-Strom mit dem gasförmigen Rohproduktstrom (201) in Kontakt zu bringen, bevorzugt in diesen einzudüsen. Dies geschieht bevorzugt durch Einleiten des Kondensatstroms (208) in den Sumpf der Waschkolonne (A22), in dem sich die flüssige Phase (215) befindet. Der Waschkolonne wird dann ein vereinigter Quenchflüssigkeits-Strom entnommen, der in Schritt (i) eingesetzt wird. In dieser Ausführungsform kann die Pumpe (P23) entfallen.

**Schritt (vi)** des erfindungsgemäßen Verfahrens, die Aufarbeitung der in Schritt (i) erhaltenen Flüssigphase (202), kann nach einem beliebigen aus dem Stand der Technik bekannten Verfahren erfolgen. Ein teilweises Zurückführen des Stroms (202) zur Quenchzone (A21) entfällt im erfindungsgemäßen Verfahren; auf die Kreislaufpumpe (P11) und den potenziell versclnnutzungsanfälligen Quenchekühler (W11) kann somit verzichtet werden. Aufgrund des nur einmaligen Durchlaufens der Quenche wird das Produkt kürzer thermisch belastet, was die Nebenproduktbildung reduziert. Da der Quenchesumpf in Fig. 1 gleichzeitig als Pumpvorlage für die große Kreislaufpumpe (P11) fungiert, kann der Hold-Up des Quenchesumpfes im Verfahren gemäß Fig. 2 ohne Kreislaufpumpe oder nur mit einer wesentlich kleineren Produktaustragspumpe kleiner dimensioniert werden, was die Verweilzeit des Quenchesumpfroduktes (202) und damit die Nebenproduktbildung weiter reduziert. Der flüssige rohe Produktgemischstrom (202) enthält im Wesentlichen Isocyanat, Lösungsmittel (aus der Quenchflüssigkeit) sowie einen geringen Anteil an nicht umgesetztem Phosgen. Dieser flüssige rohe Produktgemischstrom wird anschließend einer destillativen, im Allgemeinen mehrstufigen, Aufarbeitung zugeführt, wobei gelöstes Phosgen sowie das Lösungsmittel abgetrennt werden. Diese destillative Aufarbeitung des rohen Isocyanats kann nach allgemein bekannten Methoden erfolgen. Beispiele sind in EP-A-1 413 571, US 2003/0230476 A1 (TDI), und EP 0289 840 B1 (HDI, IDPI und H12-MDI) beschrieben.

Das erfindungsgemäße Verfahren weist darüber hinaus noch die folgenden Vorteile gegenüber dem Stand der Technik auf:
Im Unterschied zum Stand der Technik (Fig. 1) erhält man in im erfindungsgemäßen Verfahren (Fig. 2) einen zusätzlichen Freiheitsgrad: Wie zuvor erwähnt, muss beim Verfahren nach dem Stand der Technik die Wahl der Temperatur der Stoffströme am Austritt der Quenchzone unter Abwägung von geringer thermischer Produktbelastung und geringem Lösungsmittel- und Isocyanatanteil im zu reinigenden und zu rezyklierenden Brüdenstrom einerseits *(niedrige Temperatur ist positiv)* und geringer Carbaminsäurechlorid-Bildung und geringem Energiebedarf in der Produktaufreinigung andererseits *(hohe Temperatur ist positiv)* erfolgen. Im erfindungsgemäßen Verfahren können die Quenche-Sumpftemperatur und die Waschkolonnen-Eintrittstemperatur dagegen unabhängig voneinander gewählt werden. So wird die Carbaminsäurechlorid-Bildung verringert und in der Produktaufarbeitung Energie gespart (Vorwärmen des Destillationskolonnenzulaufs), während gleichzeitig die thermische Produktbelastung aufgrund des nur einmaligen Durchlaufs durch die Quenchzone zeitlich verringert wird und der höhere Lösungmittel- und Isocyanatanteil im Brüdenstrom nicht zu einer größeren, aufwändigeren Waschkolonnen führt.

Bei gleicher Quenchesumpftemperatur wird die gemäß Fig. 2 reduzierte Kondensationsleistung des Kondensators (W22) zwar "nur" in den neuen Kondensator (W23) "verschoben"; aufgrund des höheren prozessseitigen Temperaturniveaus in (W23) kann der Apparat jedoch kleiner gebaut und besser für eine wärmeintegrative Verschaltung von Prozessströmen verwendet werden. Durch das höhere prozessseitige Temperaturniveau ist auch eine teilweise Nutzung der Kühlleistung zur Niederdruckdampferzeugung möglich. Ähnliches gilt für den Quenchekühler: Die Kühlleistung des entfallenden Quenchekühlers (W11) wird zwar "nur" in den neuen Kondensator (W23) "verschoben", wegen des höheren Wärmeübergangs aufgrund von prozessseitiger Kondensation und prozessseitig höherem Temperaturverlauf in (W23) kann der Apparat jedoch ebenfalls kleiner gebaut und besser für eine wärmeintegrative Verschaltung von Prozessströmen verwendet werden.

Im Verfahren gemäß Fig. 1 bestimmt die Menge an Waschflüssigkeit (110) den Lösungsmittelgehalt des flüssigen rohen Produktgemischs im Quenchesumpf (102). Eine geringere Lösungsmittelkonzentration verringert den Produktaufreinigungsaufwand und ist daher energetisch und apparativ vorteilhaft. Die zum Erzielen einer ausreichenden Isocyanatwäsche in der Waschkolonne (A12) nötige Lösungsmittelmenge begrenzt die wünschenswerte Absenkung des Lösungsmittelgehalts im flüssigen Produktgemisch (103) jedoch nach unten. Diese Limitierung wird insbesondere bei geringen Lösungsmittelgehalten im flüssigen Produktgemisch (103) wirksam. Im erfindungsgemäßen Verfahren gemäß Fig. 2 werden weit niedrigere Lösungsmittelgehalte im flüssigen rohen Produktgemisch möglich, da der Isocyanatgehalt im Eintritt zur Waschkolonne bereits wesentlich niedriger ist und daher weniger Waschflüssigkeit zum Zurückhalten des Isocyanats benötigt wird, als gemäß Stand der Technik (Fig. 1).

Um die Quenche-Sumpftemperatur gemäß dem Stand der Technik (Fig. 1) konstant zu halten, wird üblicherweise die Eintrittstemperatur des aus dem Quenchesumpf auf die Quenche zurückgeführten flüssigen rohen Produktgemischs aktiv geregelt. Insbesondere bei Lastwechseln der Reaktionszone kommt es zu unerwünschten Schwankungen der Quenche-Sumpftemperatur. In der erfindungsgemäßen Prozessführung gemäß Fig. 2 steigt bei steigender Quenche-Sumpftemperatur entsprechend dem Dampf-Flüssig-Gleichgewicht der Dampfförmig aus der Quenche abgezogene Brüdenstrom. Bei unveränderter Kondensationstemperatur in (W23) steigt damit automatisch die flüssig abgezogene Kondensattnenge, die als Quencheflüssigkeit zurückgeführt wird und ohne aktive Regelung der Quenche-Sumpftemperatur-Erhöhung entgegen wirkt. Das erfindungsgemäße Verfahren weist somit ein vorteilhaftes, sich (auf Grund der Thermodynamik) selbst dämpfendes dynamisches Betriebsverhalten auf.

### Beispiele

Die Verfahren gemäß Fig. 1 (Vergleich - Beispiel 1) und Fig. 2 (erfindungsgemäß - Beispiel 2) wurden mittels Prozess-Simulation nachgestellt. In beiden Fällen wurde am Eintritt der (A11) bzw. (A21) vorgeschalteten Reaktionszone (in den Abbildungen nicht gezeigt), deren einziger Austrittsstrom der gasförmige Rohproduktstrom (101) bzw. (201) ist, von einem gasförmigen Eduktgemisch mit folgender Zusammensetzung der wesentlichen Bestandteile (untergeordnete Anteile an Nebenprodukten vernachlässigt) ausgegangen:

| | |
|---|---|
| Phosgen | 76 Massen-% |
| TDA-Isomere | 24 Massen-% |
| Temperatur | 400 °C |
| Druck | 1400 mbar (abs) |

Das Volumen des Brüdenstroms zur Waschkolonne (207) war in Beispiel 2 55 % kleiner als als in Beispiel 1 (106), und die Waschkolonne in Beispiel 2 (A22) konnte mit 30 % geringerem Durchmesser als in Beispiel 1 (A12) ausgeführt werden.

Der Brüdenstrom (207) zur Waschkolonne (A22) enthielt eine etwa um den Faktor 1000 geringere Isocyanatmenge als der Brüdenstrom (106) zur Waschkolonne (A12). Die für den Betrieb der Waschkolonne (A22) nötige Waschflüssigkeitsmenge und damit die Lösungsmittelkonzentration im Rohwaregemisch (202) konnte dementsprechend im Vergleich zu Beispiel 1 wesentlich verringert werden, ohne den Isocyanatanteil im aufzuarbeitenden Phosgen-Chlorwasser-Gemisch (214) gegenüber (114) zu erhöhen.

Im erfindungsgemäßen Beispiel gemäß Fig. 2 wurde die für den betrachteten Prozessabschnitt notwendige Wärmetauscherfläche (Summe der Flächen von W23 und W22; als Maß für die Apparatekosten) im Vergleich zu Beispiel 1 (Fig. 1, Summe der Flächen von W11 und W12) um 40 % reduziert: Zwar war die notwendige Kühlleistung beider Varianten vergleichbar groß, aber auf Grund des in W23 prozessseitig vorliegenden Kondensationsvorgangs war der Wärmeübergangskoeffizient größer als in W11, in dem prozessseitig ein flüssiger Strom vorliegt. Kühlmittelseitig wurde dabei in allen betrachteten Wärmetauschern von einem flüssigen Kühlmittel ausgegangen.

Im erfindungsgemäßen Beispiel (Fig. 2) betrug die nötige Pumpenkapazität von P23 nur 15 % der entsprechenden Pumpe P11 gemäß Beispiel 1 (Fig. 1), was unter anderem bezüglich der Investitions- und Betriebskosten vorteilhaft ist.

## Patentansprüche

1. Verfahren zur Abtrennung eines durch Umsetzung eines primären Amins mit einem Überschuss an Phosgen in der Gasphase hergestellten Isocyanats aus dem in der Umsetzung erhaltenen gasförmigen Rohprodukt, bei dem
(i) das gasförmige Rohprodukt (201), das mindestens Isocyanat, Chlorwasserstoff und nicht umgesetztes Phosgen umfasst, durch Inkontaktbringen mit mindestens einer Quenchflüssigkeit in eine mindestens Quenchflüssigkeit und Isocyanat umfassende Flüssigphase (202) und in eine mindestens Chlorwasserstoff und Phosgen umfassende Gasphase (206) überführt wird,
(ii) die in Schritt (i) erhaltene Gasphase (206) teilweise kondensiert wird,
(iii) das in Schritt (ii) erhaltene Kondensat (208) als Quenchflüssigkeit in Schritt (i) eingesetzt wird,
(iv) die in Schritt (ii) nicht kondensierten Anteile (207) der Gasphase (206) zumindest teilweise unter Erhalt einer flüssigen Phase (215) verflüssigt werden,
(v) die in Schritt (iv) erhaltene flüssige Phase (215) ebenfalls als Quenchflüssigkeit in Schritt (i) eingesetzt wird, und
(vi) die in Schritt (i) erhaltene Flüssigphase (202) ohne vorherigen Einsatz als Quenchflüssigkeit zum reinen Isocyanat aufgearbeitet wird.

2. Verfahren nach Anspruch 1, bei dem in Schritt (ii) ein Kondensator (W23) eingesetzt wird, in dem die gasförmige Phase und die flüssige Phase prozessseitig im Gleichstrom geführt werden.

3. Verfahren nach Anspruch 1 oder 2, bei dem die zumindest teilweise Verflüssigung der in Schritt (ii) erhaltenen nicht kondensierten Anteile (207) der Gasphase (206) in Schritt (iv) in einer Waschkolonne (A22) mit mindestens einer Trennstufe durchgeführt wird, wobei der Waschkolonne (A22) mindestens ein Lösungsmittelstrom als Waschflüssigkeit zugeführt wird.

4. Verfahren nach Anspruch 3, bei dem der Waschkolonne (A22) ein gasförmiger Kopfstrom (212) entnommen und dieser in einem Kondensator (W22) kondensiert wird und das so erhaltene Kondensat (213) als zusätzliche Waschflüssigkeit in die Waschkolonne (A22) zurückgeführt wird.

5. Verfahren nach Anspruch 3 oder 4, bei dem der Waschkolonne (A22) genau ein Lösungsmittelstrom zugeführt wird.

6. Verfahren nach einem der Ansprüche 3 bis 5, bei dem ein Lösungsmittelstrom (210) oberhalb der Trennstufe der Waschkolonne (A22), bei Vorhandensein mehrerer Trennstufen oberhalb der obersten Trennstufe, zugeführt wird, wobei der Lösungsmittelstrom (210) das abzutrennende Isocyanat in einem Massenanteil von 0 ppm bis 100 ppm, bezogen auf die Gesamtmasse des Lösungsmittelstroms (210), enthält.

7. Verfahren nach einem der Ansprüche 3 bis 6, bei dem ein Lösungsmittelstrom (211) unterhalb der Trennstufe der Waschkolonne (A22), bei Vorhandensein mehrerer Trennstufen unterhalb der untersten Trennstufe, zugeführt wird, wobei der Lösungsmittelstrom (211) das abzutrennende Isocyanat in einem Massenanteil von 0 bis 20 Massen-%, bezogen auf die Gesamtmasse des Lösungsmittelstroms (211), enthält.

8. Verfahren nach einem der Ansprüche 1 bis 7, bei dem Schritt (ii) zur Gewinnung von Dampf genutzt wird.

9. Verfahren nach einem der Ansprüche 1 bis 8, bei dem die Quenchflüssigkeit des Schritts (iii) (208) und die Quenchflüssigkeit des Schritts (v) (215) in Schritt (i) getrennt voneinander (209, 216) mit dem gasförmigen Rohprodukt der Phosgenierung (201) in Kontakt gebracht werden.

10. Verfahren nach Anspruch 9, bei dem die Quenchflüssigkeit (216) oberhalb der Quenchflüssigkeit (209) mit dem gasförmigen Rohproduktstrom (201) in Kontakt gebracht wird.

11. Verfahren nach einem der Ansprüche 1 bis 8, bei dem die Quenchflüssigkeit des Schritts (iii) (208) und die Quenchflüssigkeit des Schritts (v) (215) vereint und in Schritt (i) gemeinsam als ein Quenchflüssigkeits-Strom mit dem gasförmigen Rohproduktstrom (201) in Kontakt gebracht werden werden.

12. Verfahren nach einem der Ansprüche 1 bis 11, bei dem
(a) ein Teil (219) des in Schritt (ii) erhaltenen Kondensats (208) oder
(b) ein Lösungsmittelstrom (220) oder
(c) Strom (a) (219) und Strom (b) (220),
zusätzlich zu der in Schritt (i) erhaltenen Gasphase (206) in den Kondensator (W23) geleitet wird/werden.

13. Verfahren nach einem der Ansprüche 1 bis 12, bei dem das Inkontaktbringen des gasförmigen Rohprodukts (201) mit Quenchflüssigkeit durch Eindüsen der Quenchflüssigkeit in das gasförmige Rohprodukt (201) erfolgt.

14. Verfahren nach einem der Ansprüche 1 bis 13, bei dem das primäre Amin ausgewählt ist aus mindestens einem Mitglied der Gruppe bestehend aus den Isomeren des Toluylendiamins, den Isomeren des Diphenylmethandiamins, 1,6-Hexamethylendiamin, den Isomeren des Isophorondiamins und den Isomeren des Diaminodicyclohexylmethans.

## Claims

1. Process for separating an isocyanate prepared by reaction of a primary amine with an excess of phosgene in the gas phase from the gaseous crude product obtained in the reaction, wherein
(i) the gaseous crude product (201), which comprises at least isocyanate, hydrogen chloride and unreacted phosgene, is converted by contacting with at least one quenching liquid into a liquid phase (202) comprising at least quenching liquid and isocyanate and into a gas phase (206) comprising at least hydrogen chloride and phosgene,
(ii) the gas phase (206) obtained in step (i) is partially condensed,
(iii) the condensate (208) obtained in step (ii) is used as the quenching liquid in step (i),
(iv) the portions (207) of the gas phase (206) that were not condensed in step (ii) are at least partially liquefied to obtain a liquid phase (215),
(v) the liquid phase (215) obtained in step (iv) is likewise used as the quenching liquid in step (i), and
(vi) the liquid phase (202) obtained in step (i) is worked up to the pure isocyanate without previously being used as quenching liquid.

2. Process according to claim 1, wherein there is used in step (ii) a condenser (W23) in which the gaseous phase and the liquid phase are guided cocurrently on the process side.

3. Process according to claim 1 or 2, wherein the at least partial liquefaction of the uncondensed portions (207) of the gas phase (206) obtained in step (ii) is carried out in step (iv) in a washing column (A22) having at least one separator stage, wherein at least one solvent stream is fed to the washing column (A22) as the washing liquid.

4. Process according to claim 3, wherein a gaseous top stream (212) is removed from the washing column (A22) and condensed in a condenser (W22), and the condensate (213) so obtained is fed back into the washing column (A22) as additional washing liquid.

5. Process according to claim 3 or 4, wherein exactly one solvent stream is fed to the washing column (A22).

6. Process according to any one of claims 3 to 5, wherein a solvent stream (210) is fed to the washing column (A22) above the separator stage, where a plurality of separator stages are present above the uppermost separator stage, wherein the solvent stream (210) comprises the isocyanate to be separated off in an amount by mass of from 0 ppm to 100 ppm, based on the total mass of the solvent stream (210).

7. Process according to any one of claims 3 to 6, wherein a solvent stream (211) is fed to the washing column (A22) beneath the separator stage, where a plurality of separator stages are present beneath the lowermost separator stage, wherein the solvent stream (211) comprises the isocyanate to be separated off in an amount by mass of from 0 to 20 % by mass, based on the total mass of the solvent stream (211).

8. Process according to any one of claims 1 to 7, wherein step (ii) is used to obtain steam.

9. Process according to any one of claims 1 to 8, wherein the quenching liquid of step (iii) (208) and the quenching liquid of step (v) (215) are brought into contact with the gaseous crude product of the phosgenation (201) separately from one another (209, 216) in step (i).

10. Process according to claim 9, wherein the quenching liquid (216) is brought into contact with the gaseous crude product stream (201) above the quenching liquid (209).

11. Process according to any one of claims 1 to 8, wherein the quenching liquid of step (iii) (208) and the quenching liquid of step (v) (215) are combined and brought into contact with the gaseous crude product stream (201) in step (i) together as a quenching liquid stream.

12. Process according to any one of claims 1 to 11, wherein
(a) a portion (219) of the condensate (208) obtained in step (ii) or
(b) a solvent stream (220) or
(c) stream (a) (219) and stream (b) (220)
is/are passed into the condenser (W23) in addition to the gas phase (206) obtained in step (i).

13. Process according to any one of claims 1 to 12, wherein the contacting of the gaseous crude product (201) with quenching liquid is carried out by injecting the quenching liquid into the gaseous crude product (201).

14. Process according to any one of claims 1 to 13, wherein the primary amine is chosen from at least one member of the group consisting of the isomers of toluylenediamine, the isomers of diphenyl-methanediamine, 1,6-hexamethylenediamine, the isomers of isophoronediamine and the isomers of diaminodicyclohexylmethane.

## Revendications

1. Procédé de séparation d'un isocyanate fabriqué par mise en réaction d'une amine primaire avec un excès de phosgène en phase gazeuse du produit brut gazeux obtenu lors de la réaction, selon lequel :
(i) le produit brut gazeux (201), qui comprend au moins l'isocyanate, du chlorure d'hydrogène et du phosgène non réagi, est transformé par mise en contact avec au moins un liquide de trempe en une phase liquide (202) comprenant au moins le liquide de trempe et l'isocyanate et en une phase gazeuse (206) comprenant au moins du chlorure d'hydrogène et du phosgène,
(ii) la phase gazeuse (206) obtenue à l'étape (i) est partiellement condensée,
(iii) le condensat (208) obtenu à l'étape (ii) est utilisé en tant que liquide de trempe à l'étape (i),
(iv) les fractions (207) non condensées à l'étape (ii) de la phase gazeuse (206) sont au moins partiellement liquéfiées avec obtention d'une phase liquide (215),
(v) la phase liquide (215) obtenue à l'étape (iv) est également utilisée en tant que liquide de trempe à l'étape (i), et
(vi) la phase liquide (202) obtenue à l'étape (i) est traitée pour obtenir l'isocyanate pur sans utilisation préalable en tant que liquide de trempe.

2. Procédé selon la revendication 1, selon lequel un condensateur (W23) est utilisé à l'étape (ii), dans lequel la phase gazeuse et la phase liquide sont mises en circulation à co-courant du côté du procédé.

3. Procédé selon la revendication 1 ou 2, selon lequel la liquéfaction au moins partielle des fractions (207) non condensées de la phase gazeuse (206) obtenues à l'étape (ii) est réalisée à l'étape (iv) dans une colonne de lavage (A22) comprenant au moins une étape de séparation, au moins un courant de solvant étant introduit dans la colonne de lavage (A22) en tant que liquide de lavage.

4. Procédé selon la revendication 3, selon lequel un courant de tête gazeux (212) est soutiré de la colonne de lavage (A22) et celui-ci est condensé dans un condensateur (W22) et le condensat (213) ainsi obtenu est recyclé en tant que liquide de lavage supplémentaire dans la colonne de lavage (A22).

5. Procédé selon la revendication 3 ou 4, selon lequel exactement un courant de solvant est introduit dans la colonne de lavage (A22).

6. Procédé selon l'une quelconque des revendications 3 à 5, selon lequel un courant de solvant (210) est introduit au-dessus de l'étape de séparation de la colonne de lavage (A22), au-dessus de l'étape de séparation la plus supérieure lorsque plusieurs étapes de séparation sont présentes, le courant de solvant (210) contenant l'isocyanate à séparer en une proportion en masse de 0 ppm à 100 ppm, par rapport à la masse totale du courant de solvant (210).

7. Procédé selon l'une quelconque des revendications 3 à 6, selon lequel un courant de solvant (211) est introduit en dessous de l'étape de séparation de la colonne de lavage (A22), en dessous de l'étape de séparation la plus inférieure lorsque plusieurs étapes de séparation sont présentes, le courant de solvant (211) contenant l'isocyanate à séparer en une proportion en masse de 0 à 20 % en masse, par rapport à la masse totale du courant de solvant (211).

8. Procédé selon l'une quelconque des revendications 1 à 7, selon lequel l'étape (ii) est utilisée pour l'obtention de vapeur.

9. Procédé selon l'une quelconque des revendications 1 à 8, selon lequel le liquide de trempe de l'étape (iii) (208) et le liquide de trempe de l'étape (v) (215) sont ms en contact séparément l'un de l'autre (209, 216) à l'étape (i) avec le produit brut gazeux de la phosgénation (201).

10. Procédé selon la revendication 9, selon lequel le liquide de trempe (216) est mis en contact au-dessus du liquide de trempe (209) avec le courant de produit brut gazeux (201).

11. Procédé selon l'une quelconque des revendications 1 à 8, selon lequel le liquide de trempe de l'étape (iii) (208) et le liquide de trempe de l'étape (v) (215) sont réunis et mis en contact ensemble à l'étape (i) en tant que courant de liquide de trempe avec le courant de produit brut gazeux (201).

12. Procédé selon l'une quelconque des revendications 1 à 11, selon lequel
(a) une partie (219) du condensat (208) obtenu à l'étape (ii) ou
(b) un courant de solvant (220) ou
(c) un courant (a) (219) et un courant (b) (220),
sont introduits dans le condensateur (W23) en plus de la phase gazeuse (206) obtenue à l'étape (i).

13. Procédé selon l'une quelconque des revendications 1 à 12, selon lequel la mise en contact du produit brut gazeux (201) avec le liquide de trempe a lieu par injection du liquide de trempe dans le produit brut gazeux (201).

14. Procédé selon l'une quelconque des revendications 1 à 13, selon lequel l'amine primaire est choisie parmi au moins un élément du groupe constitué par les isomères de toluylène-diamine, les isomères de diphénylméthane-diamine, la 1,6-hexaméthylène-diamine, les isomères d'isophorone-diamine et les isomères de diaminodicyclohexylméthane.
